# EUROPÄISCHE PATENTANMELDUNG

(11) **EP 2 409 680 A2**
(43) Veröffentlichungstag der Anmeldung: **25.01.2012**
(21) Anmeldenummer: 11159002.2
(22) Anmeldetag: 21.03.2011
(51) Int. Cl.: A61K 8/44, A61K 8/46, A61K 8/60, A61Q 19/10

(54) **Kosmetisches Körperreinigungsmittel mit nicht spürbarem Fehlen von alkoxylierten Tensiden**

(30) Priorität: 31.05.2010 DE 102010022062
(71) Anmelder: Beiersdorf AG, 20253 Hamburg (DE)
(72) Erfinder: Nilsson, Jan, 22307, Hamburg (DE); Bürger, Anette, 20457, Hamburg (DE); Fiedler, Dorothe, 22335, Hamburg (DE); Hüttl, Matthias, 22769, Hamburg (DE)

(57) **Zusammenfassung**

Körperreinigungsmittel, mit höchstens 0,1 Gew.-% alkoxylierten Tensiden (im HLB-Bereich von 13 bis 18, besonders bevorzugt LES ≤0,10%), gekennzeichnet durch einen Gehalt von
(A) 2,9 bis 6,3 Gew.-% Ammoniumlaurylsulfat,
(B) 1,1 bis 2,45 Gew.-% Cocamidopropylbetain oder 2,6 bis 7,7 Gew.-% Laurylglucosid,
(C) 2,2 bis 6,65 Gew.-% Cocoglucosid,
(D) 0,85 bis 1,8 Gew.-% Natriumcocoamphoacetat.

## Beschreibung

Die Erfindung betrifft ein transparentes oder opakes Körperreinigungsmittel, welches ≤ 0,10 % alkoxylierte Tenside enthält und im Wesentlichen frei von Verdickern und Parabenen ist.

Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und für die gewünschte Schaumregulierung.

Die hohe Reinigungsleistung tensidhaltiger Reinigungszubereitungen birgt jedoch auch eine Reihe von dermatologischen Nachteilen in sich: Bereits bei einer Reinigung der Haut mit Hilfe von Wasser - ohne Zusatz von Tensiden - kommt es zunächst zu einer Quellung der Hornschicht der Haut. Der Grad dieser Quellung hängt u. a. von der Dauer des Bades und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung und Reizung der Haut. Durch zunehmend häufigeren Kontakt mit Körperreinigungsprodukten, z. B. mit Duschgelen Schaumbädern oder Waschlotionen, kann es bei empfindlichen Hauttypen verstärkt zu Hautirritationen und Hautreizungen kommen, welche sich dann durch Juckreiz, Hautrötung, trockener Haut und Schuppenbildung auszeichnen.

Körperreinigungsprodukte enthalten Mischungen aus anionischen, amphoteren und nichtionischen Tensiden. Häufig sind die Tenside ethoxyliert und besitzen somit Polyethylenglycol (PEG) / C ₂ₙH₄ₙ₊₂ Oₙ₊₁ Einheiten. Manche Polyethylenglykole und Polyethylenglykol-Derivate können die Hautpermeabilität beeinflussen und unter bestimmten Voraussetzungen zu Hautreizungen wie Hautrötung und Juckreiz führen.

Insbesondere die auf Grund ihrer guten Schaumbildung häufig eingesetzten PEG-haltigen, auch als PEG-basiert bezeichneten, ethoxylierten Alkylpolyoxyethylensulfate, RO(CH₂CH₂O)ₙ SO₃Na , z. B. Natriumlaurethsulfat (LES) gelten als hautreizend.

Zu der Gruppe der nicht ethoxylierten anionischen Tenside ist das Ammoniumlaurylsulfat (C₁₂H₂₅NaO₄S) zugehörig.

Das Cocoamidopropylbetain und Natrium-Cocoamphoacetat gliedert sich in die Gruppe der amphoteren Tenside ein.

Lauryl Polyglucose (INCI EU)/Lauryl Glucoside (INCI USA) wie auch das Coco Glucoside sind nichtionische Tenside.

Die Alkoxy-Gruppen enthaltenden, waschaktiven Substanzen und Verdicker sind maßgeblich an der Bildung von Schaum und einer positiven Sensorik in Körperreinigungsmitteln enthalten.

Körperreinigungsprodukte sind im Sinne der Erfindung Duschbäder, Dusch- und Reinigungsgele, Handseifen sowie silikonfreie und silikonhaltige Shampoos.

Parabene sind weit verbreitete und häufig eingesetzte Konservierungsmittel, die in Lebensmitteln, Medikamenten und Kosmetika Verwendung finden. In der Regel gelten sie als gut verträglich, dennoch können sie im Einzelfall bei überempfindlichen Menschen allergische Reaktionen auslösen.

Es gibt bereits Produkte ohne Parabene und LES, wobei zum einen die Schaummenge und - struktur und zum anderen die Sensorik (Substantivität, Hautgefühl) im Vergleich zu LEShaltigen Produkten, stark beeinträchtigt wird: Die "Alverde Pflegedusche Grapefruit und Bambus", vertrieben von der Gesellschaft dm-drogerie markt enthält gemäß Deklaration auf der Verpackung die mit der INCI bezeichneten Komponenten Aqua, Ammonium Lauryl Sulfate, Lauryl Glucoside, Cocamidopropyl Betaine, Lauroyl Sarcosine, Bambusa Arundinacea Extract, Citrus Grandis Extract, Alcohol, Hydroxypropyl Guar Hydroxypropyltrimonium Chloride, Citrus Medica Limonum Oil, Parfum, Limonene. Die "Alverde Duschcreme Lavendel und Bergamotte", vertrieben von der Gesellschaft dm-Drogerie Markt enthält gemäß Deklaration auf der Verpackung die mit der INCI bezeichneten Komponenten Aqua, Coco Glucoside, Alcohol, Glycerin, Disodium Cocoylglutamate, Glycine Soja Oil, Xanthan Gum, Sodium Cocoyl Glutamate, Glyceryl Oleate, Olea Europaea Oil, Citric Acid, Sodium PCA, Parfum, Linalool, Limonene. Die "Alverde Pflegedusche Olive und Aloe Vera", vertrieben von der Gesellschaft dm-drogerie markt enthält gemäß Deklaration auf der Verpackung die mit der INCI bezeichneten Komponenten Aqua, Alcohol, Glycerin, Coco Glucoside, Xanthan Gum, Sodium Coco-Sulfate, Sodium Cocoyl Glutamate, Disodium Cocoylglutamate, Olea Europaea Extract, Aloe Barbadensis Gel, Calendula Officinalis Extract, Parfum, Limonene, Linalool, Citral. Die Gesellschaft Evonik AG hat folgende Rezepturen veröffentlicht:

| | Formula 1 | Formula 2 | Formula 3 | Formula 4 |
|---|---|---|---|---|
| Component | Weight % | Weight % | Weight % | Weight % |
| | | | | |
| Sodium Cocoamphoacetate | 6,00% | 5,00% | 7,00% | 4,80% |
| Disodium Lauryl Sulfosuccinate | | | | 3,60% |
| Sodium Lauryl Sulfate | 1,00% | 1,50% | 1,00% | |
| Lauryl Polyglucose/ Lauryl Glucoside | 2,00% | 1,00% | 2,00% | |
| Tegosoft ® PC 31 * | | 0,80% | 0,50% | |
| Cocamidopropyl Betaine | 3,00% | 2,50% | | 4,90% |
| ANTIL® HS 60 ** | 1,70% | | | |
| ANTIL ® Soft SC *** | | | | 0,50% |
| Perfume | | | | |
| Preservative | | | | |
| Water | 86,30% | 89,20% | 89,50% | 86,20% |
| | | | | |
| Viskosität (mPas) | 3000 | 2400 | 1400 | 3500 |
| pH-Wert | 5,5 | 5,5 | 5,5 | 5,5 |

| | | | | |
|---|---|---|---|---|
| * Tegosoft ® PC 31: Polyglyceryl-3-Caprate PEG-free refatting agent with excellent thickening properties. Provides a skin smoothing effect. ** ANTIL ® HS 60: Cocamidopropyl Betaine (31,5%), Glyceryl Laurate (20,0%), Sodium Chloride (5,5%), Water (43,0%) PEG-free refatting agent with good thickening properties, providing a pleasant skin feel in skin cleansing products.Contributes to improved rheology and gel strength of W/Si emulsions. *** ANTIL ® Soft SC: Sorbitan Sesquicaprylate ANTIL® Soft SC is a multifunctional natural based additive for surfactant formulas with excellent thickening and foam boosting properties, also in PEG-free systems. It improves foam creaminess and skin feel. | | | | |

Dem Stand der Technik mangelte es an Reinigungszubereitungen mit < 0,10 % ethoxylierten Tensiden und < 0,010 % Parabenen, welche sowohl bezüglich Schaummenge, Schaumstruktur, Langzeitstabilität und Viskosität marktüblichen Produkten mit ethoxilierten Tensiden entspricht oder diese übertrifft.

Überraschenderweise hat sich gezeigt, dass ein Körperreinigungsmittel mit höchstens 0,1 Gew.-% alkoxylierten Tensiden (im HLB-Bereich von 13 bis 18, besonders bevorzugt LES ≤0,10%) mit einem Gehalt von 2,9 bis 6,3 Gew.-% Ammoniumlaurylsulfat, 1,1 bis 2,45 Gew.-% Cocamidopropylbetain oder 2,6 bis 7,7 Gew.-% Laurylglucosid, 2,2 bis 6,65 Gew.-% Cocoglucosid, 0,85 bis 1,8 Gew.-% Natriumcocoamphoacetat den Nachteilen des Standes der Technik abhilft. Zusätzlich zu einer Schaumbildung und Schaumbeständigkeit konnte überraschenderweise zudem eine hervorragende Hautverträglichkeit festgestellt werden.

Antimikrobielle, konservierende Stoffe sind dazu da, das wasserhaltige Tensidprodukt während der Lagerung zu stabilisieren und um bspw. eine Verkeimung mit Bakterien, Pilzen und Hefen zu vermeiden. Die o.a. Kombination zeichnet sich dadurch aus, dass auf Parabene zur Konservierung verzichtet werden kann.

Die Stabilitätseigenschaften der Zubereitung ließen sich wie folgt bestimmen:
Als stabil wird eine Formel angesehen, wenn
   - die eingestellte Viskosität bei 25 °C, gemessen mit Rheomat R 123, proRheo, mit konstanter Drehzahl n = 62,5 min⁻¹ innerhalb von 6 Monaten 1800 mPas nicht unterschreitet und der pH-Wert konstant bleibt (Toleranz ±0,2).
   - Geruch und Aussehen sich über einen Zeitraum von 6 Monaten bei Raumtemperatur, bei 40 °C und unter Einstrahlung von Tageslicht nicht verändern.
   - ein Absinken des Opacifiers nach 6 Monaten bei 40°C nicht eintritt (optische Kontrolle).
   - die mikrobiologische Stabilität durch einen Belastungstest (nach European Pharmacopeia 6.0) erfüllt wird

Parfümöle dienen der Parfümierung des Produktes für den Verbraucher. Der evtl. Eigengeruch der Tenside und sonstigen Inhaltstoffe wird dadurch überdeckt und das angenehme Dufterlebnis und der Wiedererkennungswert für den Verbraucher gesteigert. Parfümfreie Produkte, für welche es aus diversen Gründen einen wachsenden Markt gibt, können daher nur sehr eigengeruchsarme Rezepturkomponenten enthalten. O.a. Tensidkombination zeichnet sich dadurch aus, dass sie aufgrund des geringen Eigengeruchs sowohl parfümfrei als auch mit Parfümölen < 0,70 % formuliert werden kann.

Das Reinigungsmittel kann sowohl transparent als auch opak formuliert werden. Die Zustände transparent und opak wurden anhand einer Transmissions- und Reflexionsmessungen an einem UV/VIS-Spektrometer (SPECORD 250, Analytik Jena AG, mit VIS-Integrationskugel für die Reflexionsmessung; Quarzglasküvette 1 cm) bestimmt.

Als transparent gelten Formulierungen bei einer Transmission >70% über den gesamten Wellenlängenbereich des sichtbaren Lichts (400 - 700 nm) und einem Reflexionsverhalten, das Wasser ähnelt (reflektierte Strahlung < 10 %, 400 - 700 nm), wohingegen opake Formulierungen eine Transmission < 5 % über den gesamten Wellenlängenbereich des sichtbaren Lichts (400 - 700 nm) aufweisen und das Reflexionsverhalten weißen Paraffinwachs (herkömmliches Teelicht) ähnelt (reflektierte Strahlung > 30 %, 400 - 700 nm).

Bei Körperreinigungsmitteln handelt es sich um Zubereitungen mit strukturviskosem Verhalten, welche eine Viskosität bei 25 °C von mindestens 1800 mPas aufweisen. Die Einstellung der gewünschten Viskosität der Körperreinigungsmittel kann durch den Einsatz verdickender Zusätze (Verdicker) erfolgen wie beispielsweise der Einsatz von Fettalkoholethoxylaten, Fettsäurealkylolamide, ethoxylierte Glucoseester, Polymere (z. B. Polyacrylsäure, Xanthan, Hydroxymethylcellulose) oder Natriumchlorid. Bei der o.a. Tensidmischung kann vollständig auf Verdicker verzichtet werden, (da die Viskosität über den pH-Wert einstellbar ist.

Die Viskosität wird mit dem Rheomat R 123 der Firma proRheo gemessen. Der Rheomat misst die Viskosität mit einem rotierenden Messkörper mit einer konstanten Drehzahl (n = 62,5 min⁻¹). Alle Messungen werden bei 25 °C durchgeführt. Das Gerät deckt einen Viskositätsbereich von 300 bis 288.000 mPas ab. Die hier beschriebenen Viskositätswerte werden mit dem Messkörper 1 gemessen. Die Messung dauert 30 Sekunden und der 30-Sekundenwert Wert ist angegeben.

Erfindungsgemäß bevorzugt ist es, wenn Parabene in Konzentrationen von 0 bis 0,5 Gew.-% eingesetzt werden. Weiter besonders bevorzugt ist es, wenn PEG/PEG-Derivate in Konzentrationen von höchstens 0,1 Gew.-% eingesetzt werden. Weiter besonders bevorzugt ist es, wenn alkoxylierte Verdicker und Salze zur Verdickung nicht eingesetzt werden. Weiter besonders bevorzugt ist es, wenn als weitere Komponenten zusätzlich ein pH-Puffersystem enthalten ist, ganz besonders bevorzugt ein Citratpuffer. Weiter besonders bevorzugt ist es, wenn als antimikrobielle, konservierende Stoffe Natriumbenzoat, Natriumsalicylat und/oder Methylisothiazolinon enthalten sind. Weiter besonders bevorzugt ist es, wenn physiologisch verträgliche Öle bis höchstens 0,7 Gew.-% enthalten sind. Weiter besonders bevorzugt ist es, wenn das erfindungsgemäße Mittel transparent oder opak ist, bestimmt durch Transmissions- und Reflexionsmessungen an einem UV/VIS-Spektrometer (SPECORD 250, Analytik Jena AG, mit VIS-Integrationskugel für die Reflexionsmessung; Quarzglasküvette 1 cm), wobei eine Transmission > 70 % und eine Reflexion < 10 % für transparente Formulierungen und das eine Transmission < 5 % und eine Reflexion > 30 % für opake Formulierungen über den gesamten Wellenlängenbereich von 400 bis 700 nm vorliegt. Weiter besonders bevorzugt ist es, wenn das opake Körperreinigungsmittel als weitere Komponenten Styrene und Acrylates Copolymer (2-Propensäure, 2-Methyl-, Polymer mit Ethenylbenzol, Opulyn 301, Hersteller Rohm & Haas) enthält. Weiter besonders bevorzugt ist es, wenn das erfindungsgemäße Körperreinigungsmittel bei einem pH-Wert von 5,2 bis 6,9 eine Viskosität von mindestens 1800 mPas aufweist (gemessen bei 25 °C mit Rheomat R 123, proRheo, bei konstanter Drehzahl (n = 62,5 min⁻¹), Messkörper 1. Ganz besonders bevorzugt ist es, wenn Mineralöle in Konzentrationen von 0 bis 0,1 Gew.-% enthalten sind oder sogar fehlen. Ebenfalls ganz besonders bevorzugt ist es, wenn Silikone in Konzentrationen von 0 bis 0,1 Gew.-% enthalten sind oder sogar fehlen. Die Erfindung umfasst auch die Verwendung eines erfindungsgemäßen Mittels zur kosmetischen Reinigung des Körpers und ein kosmetisches Reinigungsverfahren zur Reinigung der Haut und/oder des Haares des Menschen oder Haustiers wobei ein erfindungsgemäßes Mittels auf Haut und/oder Haar aufgetragen und anschließend abgespült wird.

Schließlich können die Körperreinigungsmittel die für solche Zubereitungen üblichen Hilfs-und Zusatzmittel in den üblichen Mengen enthalten. Solche üblichen Hilfsmittel sind beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte oder organische Lösemittel.

Um die Produktleistung der entwickelten Formulierungen im Vergleich zu marktüblichen Produkten zu testen, werden Expertengradings für die Sensorik (Hautgefühl- und Schaumeigenschaften) sowie Anwendungstests beim Verbraucher zu Hause (Home in use-Test) durchgeführt.

Für das Expertengrading werden ca. zehn geschulte Verbraucher eingesetzt, die das Reinigungsmittel gemäß einer standardisierten Methode anwenden. Dazu werden die Hände anfangs gründlich mit Wasser gewaschen. Das Testprodukt wird in einer definierten Menge auf die linke Hand appliziert und mit der rechten Hand verrieben (1-mal Reiben pro Sekunde). Linkshänder applizieren das Testprodukt auf der rechten Hand und verreiben das Testprodukt mit der linken Hand. Beim Verreiben werden Konsistenz, Verteilbarkeit und das Hautgefühl nach den Kriterien samtig, schmierig glitschig, emulgiert, gelig, ölig fettig und rutschig bewertet. Nach dem Verreiben werden die Schaummenge und die Schaumqualität anhand eines Standards bewertet (Der Standard ist fotographisch abgebildet (Fotographien der eingeseiften Hände). Zum Schluss beurteilt man die Abwaschzeit, das Hautgefühl im feuchten Zustand und nach dem Abtrocknen (nach einer definierten Zeit nach dem Abtrocknen).

Die Schaumbildung und das Hautgefühl für die hier beschriebene Reinigungsformulierung wurden im Expertengrading wie folgt bewertet:
- Die Formel schäumt gut auf und entwickelt dabei einen cremig feinen Schaum, der über einen längeren Zeitraum bestehen bleibt.
- Die Formel lässt sich gut verteilen.
- Die Formel entspricht den Anforderungen von PEG-haltigen marktüblichen Duschprodukten.

Bei den Kriterien samtig, schmierig glitschig, gelartig, ölig fettig und rutschig wurde die PEG-freie Formel auf demselben Niveau bewertet, wie das PEG-haltige, marktübliche Vergleichsprodukt. Beide Formeln emulgieren beim Waschen nicht. Auch nach dem Abwaschen zeigen sich keine Unterschiede zwischen PEG-haltiger und o.a. Tensidmischung. Sie sind somit sensorisch identisch.

Bei der Schaumbildung zeigt die PEG-freie, opake Formel einen gleichen Verlauf, wie die PEG-haltige Marktformel. Die transparente PEG-freie Formel zeigt beim Schaumverhalten etwas weniger mittleren Schaum, hat dafür mehr feinen Schaum.

In einem Produktanwendungstest (Home in Use-Test) wurden das Schaumverhalten und das Hautgefühl von Verbrauchern bestätigt. Es wurden zwei Tests durchgeführt. An jedem Test haben 60 Probanden teilgenommen (Rücklauf: N= 46 bzw. N=49). Alle Verbraucher waren weiblich und im Alter von 30-45 Jahren. Die Produkte wurden semimonadisch getestet. Jeweils sieben Tage sollte jedes Produkt verwendet werden. Im Test war das hier beschriebene Körperreinigungsmittel, eine PEG-haltige marktübliche Duschformel und zwei PEG-freie Duschprodukte von Alverde.

Folgende Parameter wurden abgefragt:
- Das Produkt hat einen angenehmen Duft.
- Das Produkt lässt sich sehr gut auf der Haut verteilen.
- Das Produkt schäumt gut auf.
- Die Schaummenge ist: zu gering / genau richtig / zu hoch
- Das Produkt lässt sich gut abspülen.
- Die Haut fühlt sich nach dem Abspülen angenehm an.
- Das Produkt hat eine gute Reinigungsleistung.
- Meine Haut fühlt sich gepflegt an.
- Meine Haut fühlt sich trocken an.
- Meine Haut spannt nicht.
- Das Produkt gefällt mir insgesamt gesehen sehr gut.

Bei dem Anwendungstest zeigte sich, dass das hier beschriebene Körperreinigungsmittel auf dem gleichen Niveau des PEG-haltigen Marktstandards bewertet wurde und zum Teil auch besser als die zwei PEG-freien Duschprodukte von Alverde.

Die Hautverträglichkeit wurde sowohl *in vitro* als auch *in vivo* getestet. Als in vitro-Test wurde ein Red Blood Cell Test (RBC-Test) entsprechend dem INVITTOX Protokoll Nummer 37 durchgeführt, mit dem Ergebnis, dass die hier beschriebene Formulierung moderat reizend ist und sich damit im gleichen Verträglichkeitsbereich befindet wie marktübliche, PEG-haltige Reinigungsmittel. Im dermatologischen Anwendungstest und im verkürzten Epikutantest wurde die Hautverträglichkeit auch in vivo nachgewiesen.

### Beispielrezepturen

### 1. Transparentes Duschgel

| Component | Weight % |
|---|---|
| Water | 88,1535 |
| Ammonium Lauryl Sulfate | 4,200 |
| Coco Glucoside | 3,100 |
| Cocamidopropyl Betaine | 1,630 |
| Sodium Cocoamphoacetate | 1,200 |
| Fragrance | 0,500 |
| Sodium Benzoate | 0,450 |
| Sodium Salicylate | 0,400 |
| Methylisothiazolinone | 0,090 |
| Citric Acid | 0,250 |
| Diammonium Citrate | 0,025 |
| Polyquaternium-7 (optional) | 0,0015 |

| | |
|---|---|
| pH-Wert: 5,8 Viskosität: = 5500 mpas | |

### 2. Opakes Duschgel

| Component | Weight % |
|---|---|
| Water | 87,1535 |
| Ammonium Lauryl Sulfate | 4,200 |
| Coco Glucoside | 3,100 |
| Cocamidopropyl Betaine | 1,630 |
| Sodium Cocoamphoacetate | 1,200 |
| Styrene/Acrylates Copolymer (Opacifier) | 1,000 |
| Fragrance | 0,500 |
| Sodium Benzoate | 0,450 |
| Sodium Salicylate | 0,400 |
| Citric Acid | 0,250 |
| Diammonium Citrate | 0,025 |
| Polyquaternium-7 (optional) | 0,0015 |

| | |
|---|---|
| pH-Wert: 5,4 Viskosität: = 3500 mpas | |

### 3. Transparentes Duschgel

| Component | Weight % |
|---|---|
| Water | 87,2035 |
| Ammonium Lauryl Sulfate | 4,200 |
| Coco Glucoside | 3,100 |
| Lauryl Polyglucose | 2,580 |
| Sodium Cocoamphoacetate | 1,200 |
| Fragrance | 0,500 |
| Sodium Benzoate | 0,450 |
| Sodium Salicylate | 0,400 |
| Methylisothiazolinone | 0,090 |
| Citric Acid | 0,250 |
| Diammonium Citrate | 0,025 |
| Polyquaternium-7 (optional) | 0,0015 |

| | |
|---|---|
| pH-Wert: 5,8 Viskosität: = 3000 mpas | |

### 4. Transparentes Duschgel

| Component | Weight % |
|---|---|
| Water | 84,6335 |
| Ammonium Lauryl Sulfate | 4,200 |
| Coco Glucoside | 3,100 |
| Lauryl Polyglucose | 5,150 |
| Sodium Cocoamphoacetate | 1,200 |
| Fragrance | 0,500 |
| Sodium Benzoate | 0,450 |
| Sodium Salicylate | 0,400 |
| Methylisothiazolinone | 0,090 |
| Citric Acid | 0,250 |
| Diammonium Citrate | 0,025 |
| Polyquaternium-7 (optional) | 0,0015 |

| | |
|---|---|
| pH-Wert: 5,8 Viskosität: = 6800 mpas | |

### 5. Opakes Duschgel

| Component | Weight % |
|---|---|
| Water | 81,0835 |
| Ammonium Lauryl Sulfate | 4,200 |
| Coco Glucoside | 3,100 |
| Lauryl Polyglucose | 7,700 |
| Sodium Cocoamphoacetate | 1,200 |
| Styrene/Acrylates Copolymer (Opacifier) | 1,000 |
| Fragrance | 0,500 |
| Sodium Benzoate | 0,450 |
| Sodium Salicylate | 0,400 |
| Methylisothiazolinone | 0,090 |
| Citric Acid | 0,250 |
| Diammonium Citrate | 0,025 |
| Polyquaternium-7 (optional) | 0,0015 |

| | |
|---|---|
| pH-Wert: 5,8 Viskosität: = 5400 mpas | |

## Patentansprüche

1. Körperreinigungsmittel, mit höchstens 0,1 Gew.-% alkoxylierten Tensiden (im HLB-Bereich von 13 bis 18, besonders bevorzugt LES ≤ 0,10 %), **gekennzeichnet durch** einen Gehalt von
(A) 2,9 bis 6,3 Gew.-% Ammoniumlaurylsulfat,
(B) 1,1 bis 2,45 Gew.-% Cocamidopropylbetain oder 2,6 bis 7,7 Gew.-% Laurylglucosid,
(C) 2,2 bis 6,65 Gew.-% Cocoglucosid,
(D) 0,85 bis 1,8 Gew.-% Natriumcocoamphoacetat.

2. Körperreinigungsmittel nach Anspruch 1, **dadurch gekennzeichnet, dass** Parabene in Konzentrationen von 0 bis 0,5 Gew.-% eingesetzt werden.

3. Körperreinigungsmittel nach Anspruch 1 und 2, **dadurch gekennzeichnet, dass** PEG/PEG-Derivate in Konzentrationen von höchstens 0,1 Gew.-% eingesetzt werden.

4. Körperreinigungsmittel nach Anspruch 1 bis 3, **dadurch gekennzeichnet, dass** alkoxylierte Verdicker und Salze zur Verdickung nicht eingesetzt werden.

5. Körperreinigungsmittel nach Anspruch 1 bis 4, **dadurch gekennzeichnet, dass** zusätzlich ein pH-Puffersystem enthalten ist.

6. Körperreinigungsmittel nach Anspruch 1 bis 5, **dadurch gekennzeichnet, dass** als antimikrobielle, konservierende Stoffe Natriumbenzoat, Natriumsalicylat und/oder Methylisothiazolinon enthalten sind.

7. Körperreinigungsmittel nach Anspruch 1 bis 6, **dadurch gekennzeichnet, dass** physiologisch verträgliche Öle bis höchstens 0,7 Gew.-% enthalten sind.

8. Mittel, nach einem der vorangehenden Patentansprüche **dadurch gekennzeichnet, dass** es transparent oder opak ist, bestimmt durch Transmissions- und Reflexionsmessungen an einem UV/VIS-Spektrometer (SPECORD 250, Analytik Jena AG, mit VIS-Integrationskugel für die Reflexionsmessung; Quarzglasküvette 1 cm), wobei eine Transmission >70 % und eine Reflexion <10 % für transparente Formulierungen und das eine Transmission <5 % und eine Reflexion >30 % für opake Formulierungen über den gesamten Wellenlängenbereich von 400 bis 700 nm vorliegt.

9. Opakes Körperreinigungsmittel, nach einem der vorangehenden Patentansprüche, **dadurch gekennzeichnet, dass** als weitere Komponenten Styrene und Acrylates Copolymer (2-Propensäure, 2-Methyl-, Polymer mit Ethenylbenzol, Opulyn 301, Hersteller Rohm & Haas) enthalten ist.

10. Körperreinigungsmittel nach Anspruch 1 bis 9, **dadurch gekennzeichnet, dass** es bei einem pH-Wert von 5,2 bis 6,9 eine Viskosität von mindestens 1800 mPas aufweist (gemessen bei 25 °C mit Rheomat R 123, proRheo, bei konstanter Drehzahl (n = 62,5 min⁻¹), Messkörper 1.

11. Mittel nach Anspruch 1 bis 10, **dadurch gekennzeichnet, dass** Mineralöle in Konzentrationen von 0 bis 0,1 Gew.-% enthalten sind, besonders bevorzugt fehlen.

12. Mittel nach Anspruch 1 bis 11, **dadurch gekennzeichnet, dass** Silikone in Konzentrationen von 0 bis 0,1 Gew.-% enthalten sind, besonders bevorzugt fehlen.

13. Verwendung eines Mittels nach einem der vorangehenden Patentansprüche zur kosmetischen Reinigung des Körpers.

14. Kosmetisches Reinigungsverfahren zur Reinigung der Haut und/oder des Haares des Menschen oder Haustiers, wobei ein Mittel nach einem der Patentansprüche 1 bis 12 auf Haut und/oder Haar aufgetragen und anschließend abgespült wird.
